# EUROPEAN PATENT APPLICATION

(11) **EP 4 809 942 A1**
(43) Date of publication of application: **23.09.2026**
(21) Application number: 24924856.8
(22) Date of filing: 15.02.2024
(51) Int. Cl.: A61B 17/29, A61B 17/062

(54) **SURGICAL INSTRUMENT**

(71) Applicant: Daiya Seiki Co. Ltd., Okaya-shi, Nagano 394-0084 (JP)
(72) Inventor: OMURA, Kazuhiro, Tokyo 154-0004 (JP); KOBAYASHI, Michito, Okaya-shi, Nagano 394-0084 (JP)
(74) Representative: Prüfer & Partner mbB Patentanwälte · Rechtsanwälte
(86) International application number: PCT/JP2024/005235
(87) International publication number: WO 2025/173162

(57) **Abstract**

To provide a surgical instrument in which a clamping part can be prevented from swinging. The surgical instrument for holding objects includes a main body 10, a clamping part 20, a contact part 40, an opening/closing mechanism 30, and support parts 50 (a first support part 51 and a second support part 52). The main body 10 extends in one direction. The clamping part 20 is placed at a tip end of the main body 10 and can grasp the objects. The contact part 40 is placed at a proximal end of the main body 10. The opening/closing mechanism 30 opens and closes the clamping part 20. The support parts 50 extend in one direction. The main body 10 and each of the support parts 50 are connected and fixed to intersect in a cross shape. For this reason, the surgical instrument 100 can prevent or reduce swinging of the clamping part 20 even when a wrist of a hand holding the surgical instrument 100 rotates comparing with pliers-shaped or scissor-shaped surgical instruments.

## Description

### Technical Field

The present invention relates to a surgical instrument for clamping an object during surgery, and particularly relates to a surgical instrument favorable as a needle holder by which a needle is clamped to perform suture ligation.

### Background Art

As conventional surgical instruments for clamping an object, there are those of a pliers shape or a scissors shape. For example, a needle holder of a pliers shape is disclosed in Patent Document 1 and a medical instrument and a surgical forceps each of a scissors shape are disclosed in Patent Document 2 and Patent Document 3, respectively.

The surgical instruments described in Patent Documents 1 to 3 are each arranged with a clamping part and a holding part being pivotably coupled such that when a user grips the holding part, the clamping part closes and clamps an object and when the gripping of the holding part is released, the clamping part opens and releases the clamping of the object.

### Prior Art

### Patent Documents

Patent Document 1: Japanese Post-Grant Publication No. H6-057217
Patent Document 2: Japanese Patent Publication No. 3639632
Patent Document 3: Japanese Pre-Grant Publication No. H8-038492

### Summary of Invention

### Technical Problem

However, in a state in which the surgical instrument of each of Patent Documents 1 to 3 is held, a length direction of the surgical instrument and a length direction of the forearm of the hand holding the surgical instrument intersect and therefore, the clamping part of the surgical instrument is disposed at a position separated from an axis extending along the length direction of the forearm. There is thus a problem in that, when the wrist of the hand holding the surgical instrument rotates, the clamping part of the surgical instrument swings easily and it is therefore difficult to perform operation of the surgical instrument such as clamping a needle with the clamping part of the surgical instrument and performing suture ligation while viewing an image of an endoscope, etc.

The present invention thus solves the above problem and a technical problem thereof is to provide a surgical instrument with which a clamping part of the surgical instrument is prevented from swinging and operability is improved.

### Solution to Problem

A surgical instrument of the present invention for solving the above technical problem is a surgical instrument for clamping objects and includes a main body extending in one direction, a clamping part which is placed at a tip end of the main body and which can grasp the objects, a contact part placed at a proximal end of the main body, an opening/closing mechanism opening and closing the clamping part, and support parts extending in the one direction. The main body and each of the support parts are connected and fixed to intersect in a cross shape.

According to this invention, the surgical instrument for clamping objects includes a main body extending in one direction, a clamping part which is placed at a tip end of the main body and which can grasp the objects, a contact part placed at a proximal end of the main body, an opening/closing mechanism opening and closing the clamping part, and support parts extending in one direction. The main body and the support parts are connected and fixed to intersect in a cross shape. For this reason, a length direction of the surgical instrument is made parallel or approaches being parallel to a length direction of a forearm of a hand holding the surgical instrument, and the clamping part is placed on an axis extending along the length direction of the forearm or approaches the axis when the surgical instrument is held with the contact part being contacted on a palm of the hand and fingers being latched on the support parts. Therefore, swinging of the clamping part can be prevented or reduced even when a wrist of the hand holding the surgical instrument rotates around the axis, in comparison to a surgical instrument of a pliers shape or a scissors shape.

In the present invention, preferably, the opening/closing mechanism includes an operating part, and the operating part is connected to the main body, and rotates around an axis perpendicular to a length direction of the main body, and reciprocates between a tip end side and a proximal end side of the length direction of the main body. According to this invention, the opening/closing mechanism includes an operating part, and the operating part is connected to the main body, and rotates around an axis perpendicular to a length direction of the main body, and reciprocates between a tip end side and a proximal end side of the length direction of the main body. For this reason, swinging of the clamping part due to a reciprocation of the operating part can be suppressed comparing to reciprocating in a direction perpendicular to the length direction of the main body.

In the present invention, preferably, the clamping part opens when the operating part of the opening/closing mechanism rotates to the tip end side of the main body, and the clamping part closes when the operating part of the opening/closing mechanism rotates to the proximal end side of the main body. According to this invention, the clamping part opens when the operation part of the opening/closing mechanism rotates to the tip end side of the main body, and the clamping part closes when the operating part of the opening/closing mechanism rotates to the proximal end side of the main body. For this reason, the clamping part closes when a finger is flexed and the operating part rotates to the proximal end side of the main body in the state of holding the surgical instrument. Therefore, the clamping part can be retained easily in the closed state in comparison to a case where the clamping part closes when the finger is straightened and the operating part rotates to the tip end side of the main body.

In the present invention, preferably, the opening/closing mechanism further includes a lock mechanism, and the lock mechanism retains the clamping part in a closed state and releases the retention. According to this invention, the opening/closing mechanism further includes a lock mechanism, and the lock mechanism retains the clamping part in a closed state and releases the retention. For this reason, the clamping part can retain in the closed state without continuing to apply a force to the opening/closing mechanism.

In the present invention, preferably, the lock mechanism retains the operating part of the opening/closing mechanism in a rotated position toward the proximal end side of the main body and releases the retention. According to this invention, the lock mechanism retains the operating part of the opening/closing mechanism in a rotated position toward the proximal end side of the main body and releases the retention. For this reason, the clamping part can retain in the closed state without continuing to apply a force to the operating part.

In the present invention, preferably, the support parts make of two parts which are a first support part and a second support part, and the first support part and the second support part are placed at an interval in the length direction of the main body. According to this invention, the support parts make of two parts which are a first support part and a second support part, and the first support part and the second support part are placed at an interval in the length direction of the main body. For this reason, the holding of the surgical instrument can stabilize due to the fact that portions supported by a hand can increase comparing to a case where a support part make of a single part.

In the present invention, preferably, the surgical instrument further includes a coupling part. The coupling part is arranged such that the coupling part and the contact part can be connected successively to the proximal end of the main body, and such that the contact part can be connected directly to the proximal end of the main body without using the coupling part.

According to this invention, the surgical instrument further includes a coupling part. The coupling part is arranged such that the coupling part and the contact part can be connected successively to the proximal end of the main body, and such that the contact part can be connected directly to the proximal end of the main body without using the coupling part. For this reason, a length in the length direction of the surgical instrument can be adjusted to extend and contract. Here, the connection of the proximal end of the main body and the coupling part, the connection of the coupling part and the contact part, and the connection of the proximal end of the main body and the contact part may be a screw connection, respectively.

In the present invention, preferably, the main body and the contact part are formed integrally. According to this invention, the main body and the contact part are formed integrally. For this reason, it is preventable that the contact part detaches from the main body and drops into the body of a patient during surgery.

### Advantageous Effects of Invention

As described above, according to the present invention, an excellent effect of being able to prevent the clamping part of the surgical instrument from swinging can be exhibited.

### Brief Description of Drawings

[FIG. 1] is a schematic perspective view showing a surgical instrument of an embodiment according to the present invention.
[FIG. 2] is front view of the surgical instrument of the present embodiment.
[FIG. 3] is a back view of the surgical instrument of the present embodiment.

[FIG. 4] is a right side view of the surgical instrument of the present embodiment.
[FIG. 5] is a left side view of the surgical instrument of the present embodiment.
[FIG. 6] is a plan view of the surgical instrument of the present embodiment.
[FIG. 7] is a bottom view of the surgical instrument of the present embodiment.
[FIG. 8] is a schematic perspective view showing a state in which the surgical instrument of the present embodiment is opened.
[FIG. 9] is an exploded perspective view of the surgical Instrument of the present embodiment.
[FIG. 10](a) is a schematic front view showing a state in which an opening/closing mechanism of the present embodiment is opened and FIG. 10(b) is a schematic front view showing a state in which the opening/closing mechanism is closed.
[FIG. 11](a) is a schematic view showing a state in which the surgical instrument of the present embodiment is held and FIG. 11(b) is a schematic view showing a state in which the wrist is rotated while the surgical instrument is held.
[FIG. 12](a) is a schematic view showing an operation of closing a clamping part of the surgical instrument of the present embodiment and FIG. 12(b) is a schematic view showing an operation of opening the clamping part of the surgical instrument.

### Description of Embodiments

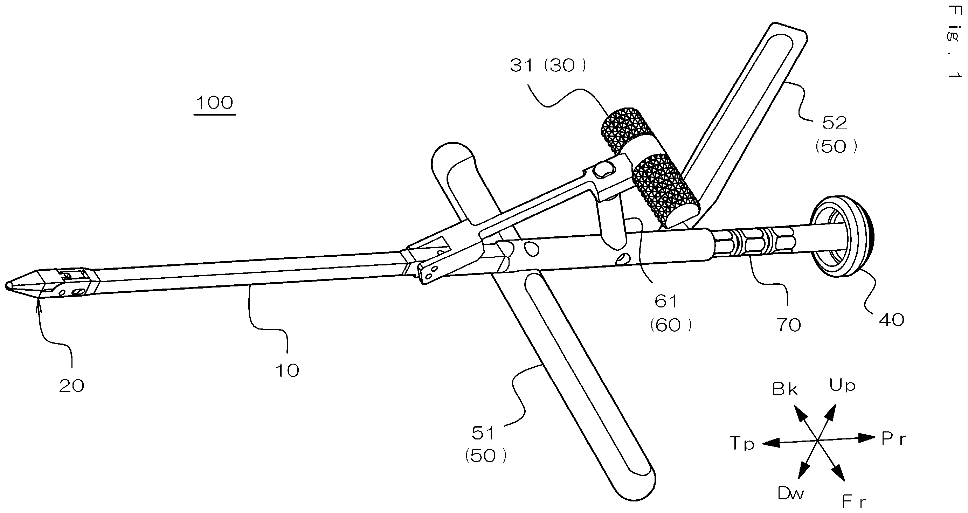

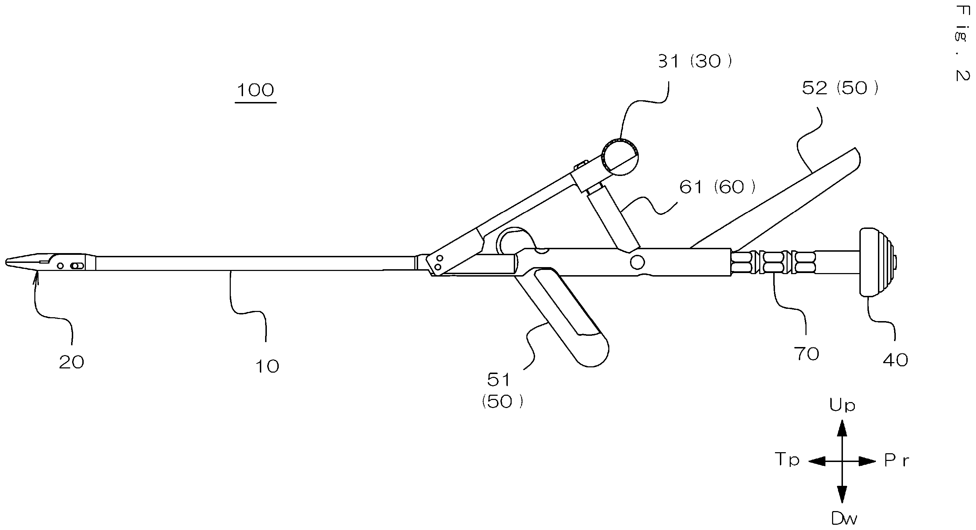

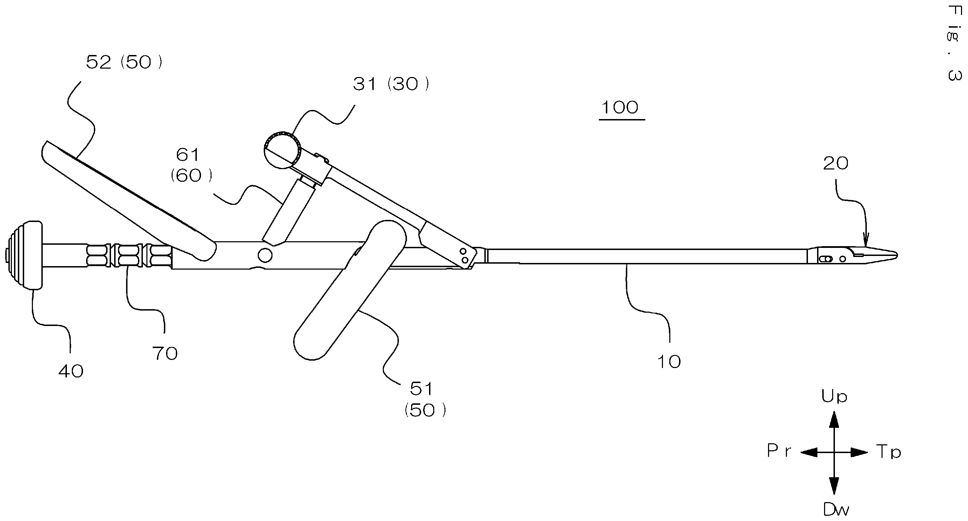

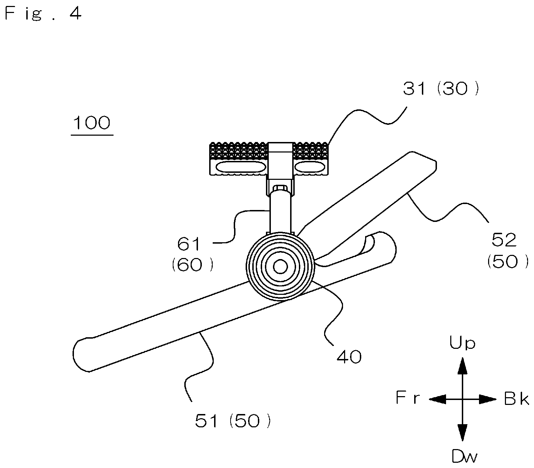

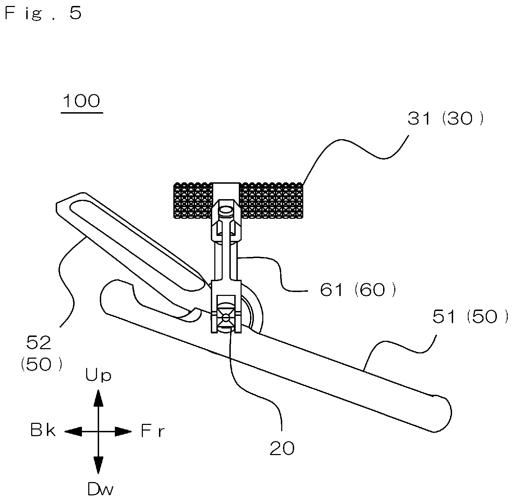

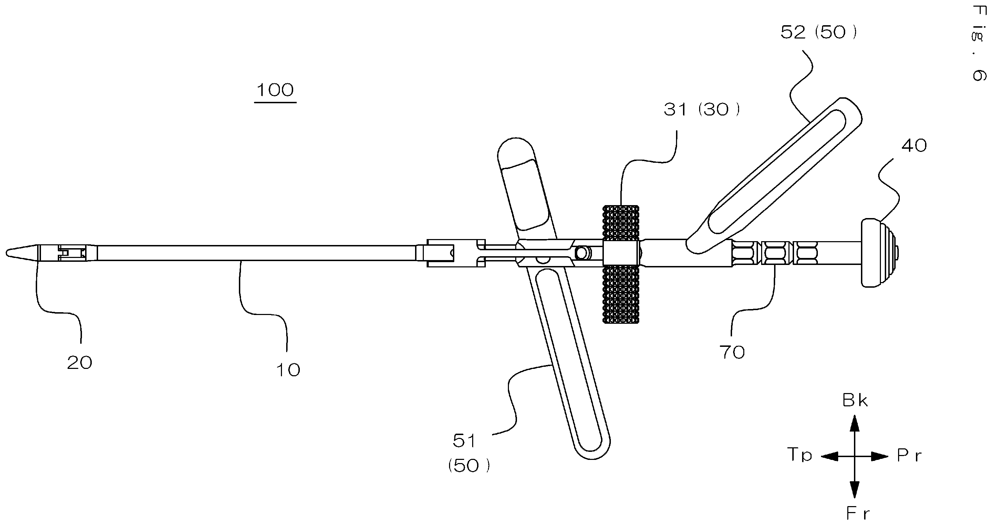

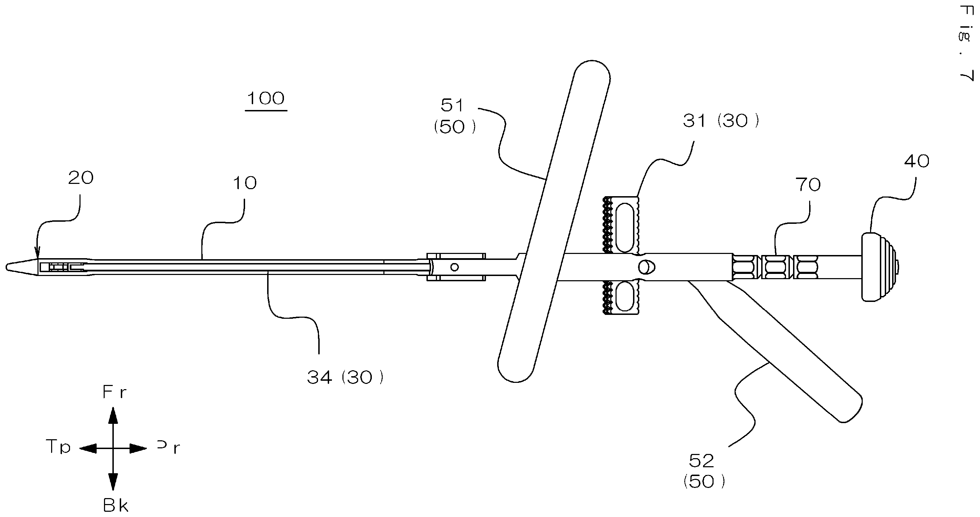

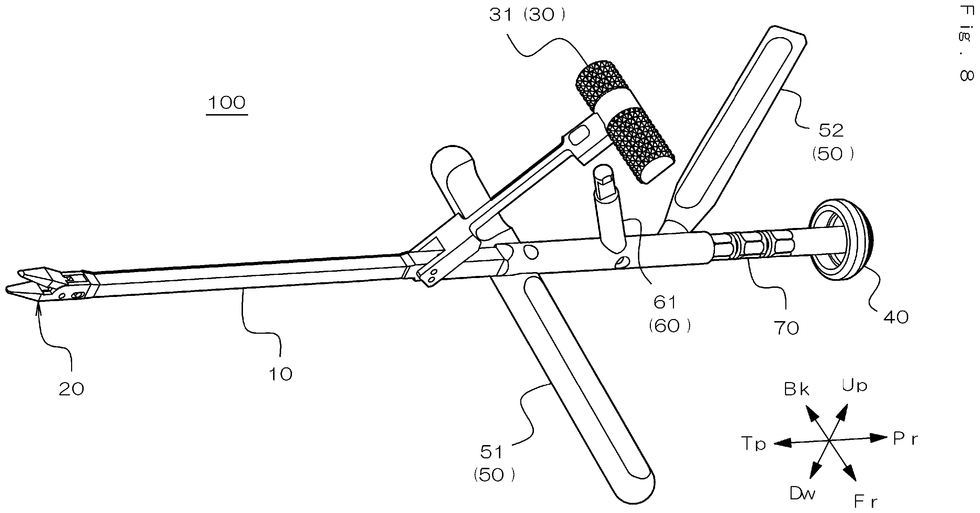

A surgical instrument of a present embodiment according to the present invention shall now be described in detail. FIG. 1 is a schematic perspective view showing a surgical instrument of an embodiment according to the present invention. FIG. 2 is a front view of the surgical instrument. FIG. 3 is a back view of the surgical instrument. FIG. 4 is a right side view of the surgical instrument. FIG. 5 is a left side view of the surgical instrument. FIG. 6 is a plan view of the surgical instrument. FIG. 7 is a bottom view of the surgical instrument. FIG. 1 to FIG. 7 show a state in which the surgical instrument is closed. FIG. 8 is a schematic perspective view showing a state in which the surgical instrument is opened. As shown in FIG. 1 to FIG. 8, the surgical instrument 100 is constituted of a metal having corrosion resistance/rust prevention property such as that of stainless steel, etc., and biocompatibility such as that of a titanium alloy, etc. This surgical instrument 100 has a main body 10, a clamping part 20, an opening/closing mechanism 30, a contact part 40, and support parts 50.

Here, in FIG. 1 to FIG. 10, a direction indicated by an arrow Up shall be an upper side (upper left side of the sheet in FIG. 1, FIG. 8, and FIG. 9, left side of the sheet in FIG. 2, FIG. 3, and FIG. 10, upper side of the sheet in FIG. 4 and FIG. 5, front side of the sheet in FIG. 6, and back side of the sheet in FIG. 7). A direction indicated by an arrow Dw shall be a lower side (lower right side of the sheet in FIG. 1, FIG. 8, and FIG. 9, right side of the sheet in FIG. 2, FIG. 3, and FIG. 10, lower side of the sheet in FIG. 4 and FIG. 5, back side of the sheet in FIG. 6, and front side of the sheet in FIG. 7). A direction indicated by an arrow Tp shall be a tip end side (lower side of the sheet in FIG. 1, FIG. 2, and FIG. 6 to FIG. 10, upper side of the sheet in FIG. 3, back side of the sheet in FIG. 4, and front side of the sheet in FIG. 5). A direction indicated by an arrow Pr shall be a proximal end side (upper side of the sheet in FIG. 1, FIG. 2, and FIG. 6 to FIG. 10, lower side of the sheet in FIG. 3, front side of the sheet in FIG. 4, and back side of the sheet in FIG. 5). A direction indicated by an arrow Fr shall be a front side (upper right of the sheet in FIG. 1, FIG. 8, and FIG. 9, front side of the sheet in FIG. 2, back side of the sheet in FIG. 3, left side of the sheet in FIG. 4 and FIG. 7, and right side of the sheet in FIG. 5 and FIG. 6). A direction indicated by an arrow Bk shall be a back side (lower left of the sheet in FIG. 1, FIG. 8, and FIG. 9, back side of the sheet in FIG. 2, front side of the sheet in FIG. 3, right side of the sheet in FIG. 4 and FIG. 7, and left side of the sheet in FIG. 5 and FIG. 6). A direction indicated by the arrow Up and the arrow Dw shall be a vertical direction. A direction indicated by the arrow Tp and the arrow Pr shall be a length direction. A direction indicated by the arrow Fr and the arrow Bk shall be a width direction. This length direction is a length direction of the surgical instrument 100 and the vertical direction and the width direction are each a direction orthogonal to the length direction. These directions indicate a relative positional relationship and do not indicate an absolute positional relationship with respect to a gravity direction.

The main body 10 of the surgical instrument 100 extends in one direction. The clamping part 20 is provided at one end part of the main body 10. This clamping part 20 is enabled to be opened and closed by the opening/closing mechanism 30 and enabled to clamp an object and release the clamping. The contact part 40 is provided at another end part of the main body 10. The support parts 50 are coupled to the main body 10 such as to intersect a length direction of the main body 10. Thereby, the surgical instrument 100 has a substantially cross shape and is enabled to be held with the contact part 40 being contacted on a palm of a hand and a finger being latched on the support parts 50.

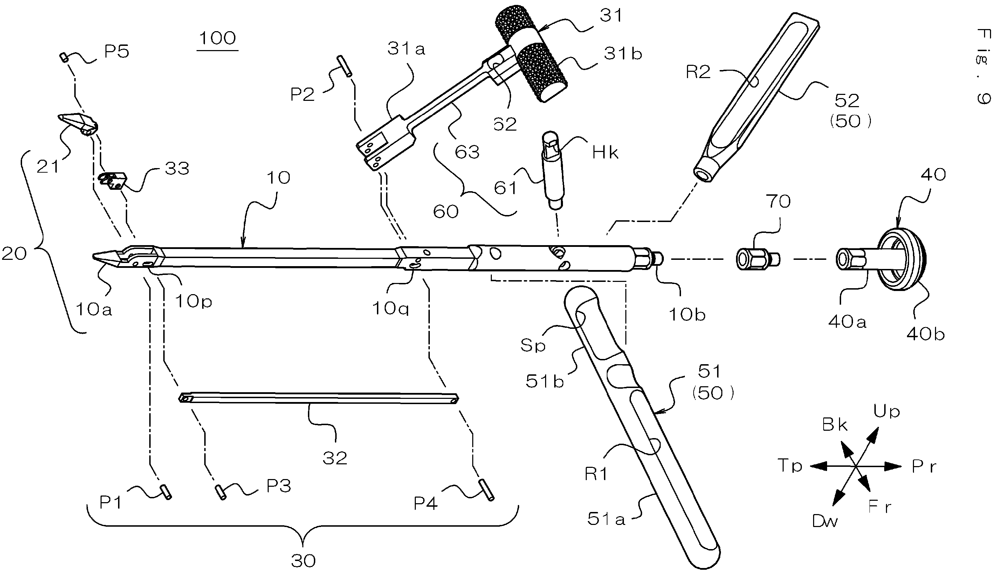

FIG. 9 is an exploded perspective view of the surgical instrument of the present embodiment. As shown in FIG. 9, more specifically, the main body 10 of the surgical instrument 100 is a rod-shaped member and extends in the length direction indicated by the arrow Tp and the arrow Pr. The clamping part 20 is provided at an end part of the main body 10 at a tip end side indicated by the arrow Tp. This clamping part 20 is constituted of a tip end part 10a of the main body 10 and a pivoting piece 21. This pivoting piece 21 is pivotably coupled to the tip end part 10a of the main body 10 by a pin P1. This pin P1 is of a circular columnar shape and is disposed at a proximal end side indicated by the arrow Pr of the pivoting piece 21 such that a portion at a tip end side of the pivoting piece 21 becomes attached and detached with respect to the tip end part 10a of the main body 10 by pivoting of the pivoting piece 21. In other words, the clamping part 20 is arranged to open and close by the pivoting of the pivoting piece 21. In the illustrated example, the pin P1 extends in the width direction indicated by the arrow Fr and the arrow Bk and the pivoting piece 21 is pivotable around an axis extending in the width direction. Also, the pivoting piece 21 and the tip end part 10a of the main body 10 are formed in shapes tapered toward the tip end side and the clamping part 20 is of a shape tapered toward the tip end side. The clamping part 20 is thereby enabled to clamp a fine object such as a needle, etc.

Next, the opening/closing mechanism 30 has an operating part 31, a shaft 32, and a joint 33. This operating part 31 is of a T shape and a finger rest part 31b of a semicircular shape is connected and fixed orthogonally to one end part of an arm part 31a extending in one direction. Another end part of the arm part 31a constitutes a tip end part of the operating part 31 and is pivotably coupled to an intermediate portion of the main body 10 by a pin P2. This pin P2 is of a circular columnar shape and extends in a direction orthogonal to the length direction of the main body 10. Thus, the operating part 31 is coupled to the main body 10 and arranged to undergo a reciprocation, which is a pivoting movement around an axis orthogonal to the length direction of the main body 10, between a tip end side and a proximal end side of the length direction of the main body 10. In the illustrated example, the pin P2 extends in the width direction, the operating part 31 extends obliquely upward to the proximal end side from the main body 10, the finger rest part 31b is disposed at an interval to the upper side indicated by the arrow Up of the main body 10, and the operating part 31 is arranged to be pivotable around the axis in the width direction. The finger rest part 31b of the operating part 31 is arranged to undergo the reciprocation between the tip end side and the proximal end side of the length direction of the main body 10. Here, a slip stopper is formed at an arcuately curved outer circumference of the finger rest part 31b for operability improvement of the operating part 31. The slip stopper is, for example, a plurality of irregularities.

The shaft 32 is a rod-shaped member extending in one direction. This shaft 32 is housed inside the main body 10 from the lower side indicated by the arrow Dw at an intermediate portion of the main body 10. The shaft 32 thus extends in the length direction like the main body 10. The joint 33 is housed inside the main body 10 from the upper side. In this state, the joint 33, the shaft 32, and the operating part 31 are aligned successively in a single column in the length direction from a tip end side toward a proximal end side. The joint 33 and the shaft 32 are pivotably coupled by a pin P3. The shaft 32 and the operating part 31 are pivotally coupled by a pin P4. The pins P3 and P4 are of circular columnar shapes and extend in a direction orthogonal to the length direction of the main body 10. In the illustrated example, the pins P3 and P4 extend in the width direction. The joint 33, the shaft 32, and the operating part 31 are thus respectively pivotable around axes extending in the width direction.

The pivoting piece 21 of the clamping part 20 is disposed adjacent to a tip end side of the joint 33 of the opening/closing mechanism 30, and the joint 33 and the pivoting piece 21 are pivotably coupled by a pin P5. In other words, the clamping part 20 and the opening/closing mechanism 30 are pivotably coupled by the pin P5. This pin 5 is of a circular columnar shape and extends in a direction orthogonal to the length direction of the main body 10. In the illustrated example, the pin P5 extends in the width direction. The pivoting piece 21 and the joint 33 are thus pivotable around an axis extending in the width direction.

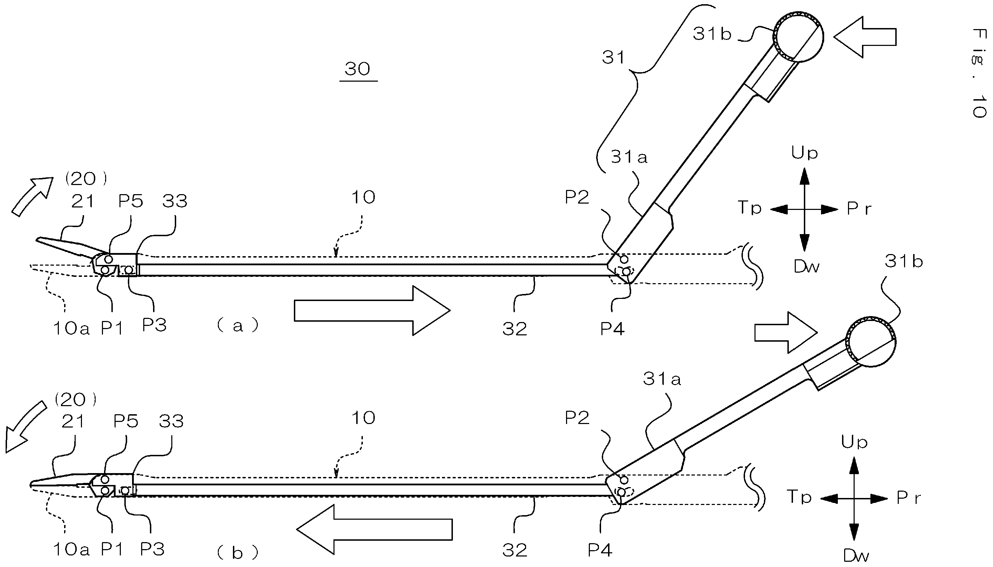

FIG. 10(a) is a schematic front view showing a state in which the opening/closing mechanism of the present embodiment is opened. FIG. 10(b) is a schematic front view showing a state in which the opening/closing mechanism is closed. As shown in FIG. 10(a) and FIG. 10(b), here, the pin P4 coupling the operating part 31 and the shaft 32 is disposed further to the lower side than the pin 2 coupling the operating part 31 and the main body 10 such that by the pivoting of the operating part 31, the pin P4 pivots around the axis of the pin P2 and the shaft 32 reciprocates in the length direction. Similarly, the pin P5 coupling the pivoting piece 21 and the joint 33 is disposed further to the upper side than the pin P1 coupling the pivoting piece 21 and the main body 10, and the joint 33 and the shaft 32 are coupled by the pin P3 such that by the reciprocation of the shaft 32, the joint 33 reciprocates in the length direction, the pivoting piece 21 pivots around the axis of the pin P1, the clamping part 20 opens and closes.

Thus, as shown in FIG. 10(a), the opening/closing mechanism 30 is arranged such that when the operating part 31 pivots to the tip end side, the shaft 32 moves to the proximal end side, the joint 33 moves to the proximal end side, the pivoting piece 21 pivots to the upper side, and the clamping part 20 opens. On the other hand, as shown in FIG. 10(b), the opening/closing mechanism 30 is arranged such that when the operating part 31 pivots to the proximal end side, the shaft 32 moves to the tip end side, the joint 33 moves to the tip end side, the pivoting piece 21 pivots to the lower side, and the clamping part 20 closes. That is, the clamping part 20 is arranged to open and close by the reciprocation of the operating part 31 between the tip end side and the proximal end side.

Returning to FIG. 9, in this state, the pins P3 and P4 are respectively engaged with slots 10p and 10q of the main body 10, and are arranged to be movable along the slots 10p and 10q. The slots 10p and 10q penetrate through the main body 10 in the width direction, the one slot 10p extends in the length direction, and the other slot 10q is curved arcuately. End parts at the proximal end side (proximal end parts) of the slots 10p and 10q correspond respectively to positions of the pins P3 and P4 when the clamping part 20 is opened and end parts at the tip end side (tip end parts) of the slots 10p and 10q correspond respectively to positions of the pins P3 and P4 when the clamping part 20 is closed. Therefore, when the clamping part 31 opens, the pins P3 and P4 respectively abut the proximal end parts of slots 10p and 10q and the pivoting of the operating part 31 stops and when the clamping part 20 closes, the pins P3 and P4 respectively abut the tip end parts of slots 10p and 10q and the pivoting of the operating part 31 stops. That is, the pins P3 and P4 and the slots 10p and 10q define a pivoting range of the operating part 31 such that breakage of the clamping part 20 and the operating part 31 can be prevented.

Here, the opening/closing mechanism 30 is provided with a lock mechanism 60. This lock mechanism 60 has a locking member 61, an opening 62, and a narrow part 63. This locking member 61 is a rod-shaped member extending in one direction, is provided with a hook Hk at one end part, and is connected and fixed to the main body 10 such as to project from an outer circumference of the main body 10. In the illustrated example, the locking member 61 is disposed at a proximal end part 10b side of the main body 10, projects obliquely upward to the tip end side from the outer circumference of the main body 10, and is provided with the hook Hk at a tip end part (upper end part) of the locking member 61.

The opening 62 is a penetrating hole formed in the operating part 31 and penetrates through from a bottom surface to an upper surface of the operating part 31. In other words, it penetrates through from a side of the operating part 31 facing the main body 10 to an opposite side with respect to the main body 10 in a direction intersecting the length direction of the main body 10. This opening 62 is arranged to enable insertion of a tip end part of the locking member 62. The arrangement is therefore such that when the operating part 31 is pivoted to the proximal end side, the tip end part of the locking member 61 is inserted into the opening 62.

The narrow part 63 is a small-diameter portion and is arranged to be elastically deformable. This narrow part 63 is provided at an intermediate portion of the arm part 31a of the operating part 31. The opening 62 is provided at the finger rest part 31b side of the arm part 31a. That is, the opening 62 is disposed further to the finger rest part 31b side than the narrow part 63. The opening 62 is thus arranged to be slightly movable in the width direction due to elastic deformation of the narrow part 63.

When the operating part 31 of the lock mechanism 60 is pivoted to the proximal end side, the tip end part of the locking member 61 is inserted into the opening 62 and the hook Hk projects to the opposite side (upper surface side) with respect to the side of the operating part 31 facing the main body 10. Thus, when in this state, the finger rest part 31b of the operating part 31 is pressed in the width direction, the narrow part 63 deforms elastically, the opening 62 moves slightly in the width direction, and the hook Hk becomes hooked to an edge part of the opening 62 or the hooking of the hook Hk is released. The lock mechanism 60 is thereby enabled to retain the operating part 31 at the proximal end side and release the retention and enabled to retain the clamping part 20 in the closed state and release the retention.

Next, the contact part 40 is provided with a hemispherical part 40b at one end of an axial part 40a extending in one direction. This axial part 40a has the same outer diameter as an outer diameter of the main body 10, and the hemispherical part 40b has a convexly curved surface of hemispherical shape having an outer dimeter greater than the outer diameter of the main body 10. The hemispherical part 40b of the contact part 40 is thus enabled to be put in contact with the palm of the hand without damaging the palm of the hand. A slip stopper is formed on this hemispherical part 40b. In the illustrated example, this slip stopper is a plurality of irregularities of concentric circular shapes.

The axial part 40a of this contact part 40 is detachably coupled to the proximal end part 10b of the main body 10 via a coupling part 70, and the hemispherical part 40b of the contact part 40 is disposed at an end part (proximal end part) at the proximal end side in the length direction of the surgical instrument 100. That is, the coupling part 70 and the contact part 40 are coupled successively to the proximal end part 10b of the main body 10, and the surgical instrument 100 extends in the length direction. In the present embodiment, the proximal end part 10b of the main body 10, the coupling part 70, and the axial part 40a of the contact part 40 are respectively screw-connected. Also, an outer circumference of each of the proximal end part 10b of the main body 10, the coupling part 70, and a tip end part (other end) of the axial part 40a of the contact part 40 is formed to a hexagonal shape in plan view. Firm tightening and easy separation from the coupled state using a spanner or other tool are thereby enabled.

The axial part 40a of the contact part 40 is arranged to be couplable to the proximal end part 10b of the main body 10, and arranged to be couplable to the coupling part 70. That is, it is made possible to successively couple the coupling part 70 and the contact part 40 to the proximal end part 10b of the main body 10 as in the illustrated example, or to directly couple the contact part 40 to the proximal end part 10b of the main body 10 without using the coupling part 70. The surgical instrument 100 is thereby enabled to be adjusted to extend and contract in the length direction.

Here, the coupling part 70 is a member of circular cylindrical shape and has the same outer diameter as the outer diameter of the main body 10. One end part of the coupling part is arranged to be couplable to the proximal end part 10b of the main body 10 and the other end part of the coupling part is arranged to be couplable to the axial part 40a of the contact part 40.

Lastly, the support parts 50 are constituted of two members and have a first support part 51 and a second support part 52. This first support part 51 and this second support part 52 are rod-shaped member extending in one direction, and are connected and fixed to the main body 10 such as to intersect the length direction of the main body 10. The first support part 51 and the second support part 52 are disposed at an interval in the length direction of the main body 10. The first support part 51 is disposed at an intermediate portion of the main body 10 and is arranged to enable latching on of fingers. The second support part 52 is disposed at the proximal end part 10b side of the main body 10 and is enabled to be disposed between fingers. In the illustrated example, the first support part 51 is disposed at a portion of the main body 10 between the operating part 31 and the locking member 61.

In a state in which the support part 50 and the main body 10 are coupled, the an intermediate portion in the length direction of the first support part 51 is connected to the main body 10, and one portion 51a and the other portion 51b of the first support part 51 protrude respectively to respective sides with the main body 10 in between. One end part of the second support part 52 is connected to the main body 10, and the second support part 52 protrudes in one direction from the main body 10. In the illustrated example, the one portion 51a of the first support part 51 protrudes obliquely downward to the front side indicated by the arrow Fr from the main body 10, and the other portion 51b of the first support part 51 protrudes obliquely upward to the back side indicated by the arrow Bk from the main body 10. The second support part protrudes obliquely upward to the back side from the main body 10.

Here, the one portion 51a of the first support part 51 has formed therein a recess part R1 extending in its length direction. Similarly, the second support part 52 has formed therein a recess part R2 extending in its length direction. Thereby, the first support part 51 and the second support part 52 are made thin in thickness and made light in weight.

Also, a notch Sp is formed in the other portion 51b of the first support part 51. This notch Sp is curved to a substantially U shape, and is arranged to enable fitting in of a finger. The other portion 51b of the first support part 51 is thus enabled to be clamped by an index finger and a middle finger with the index finger being fitted in the notch Sp and the middle finger being contacted with a portion at an opposite side with respect to the notch Sp of the other portion 51b.

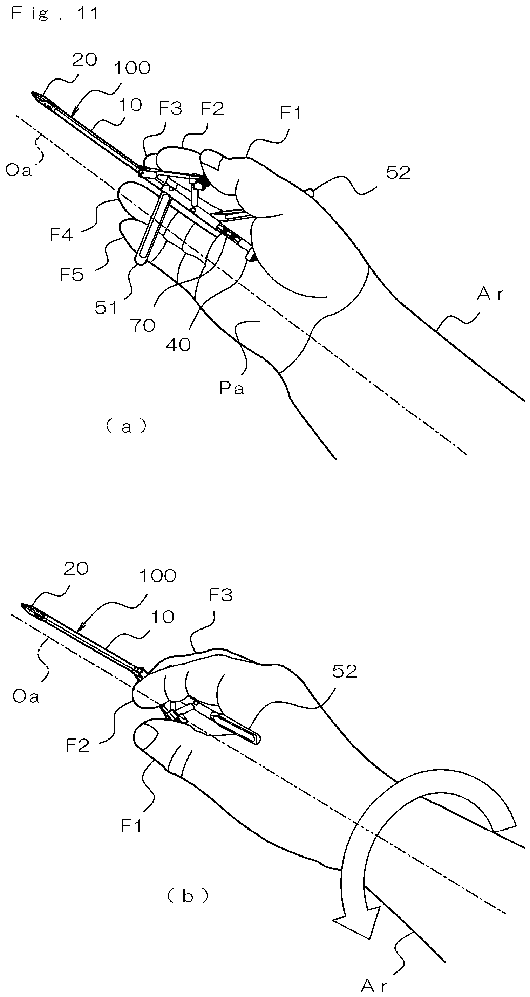

FIG. 11(a) is a schematic view showing a state in which the surgical instrument of the present embodiment is held. FIG. 11(b) is a schematic view showing a state in which the wrist is rotated while the surgical instrument of the present embodiment is held. As shown in FIG. 11(a) and FIG. 11(b), the surgical instrument 100 extends in the length direction with the clamping part 20, the main body 10, the coupling part 70, and the contact part 40 being aligned successively in one direction. The surgical instrument 100 is made to have a substantially cross shape with the first support part 51 being connected and fixed to the intermediate portion of the main body 10 such as to extend in a direction intersecting the length direction. Therefore, when the surgical instrument 100 is held with the contact part 40 being contacted with a palm Pa of the hand and fingers F2 to F5 (the index finger, the middle finger, the ring finger, and the little finger) being latched on the first support part 51, the length direction of the surgical instrument 100 is made parallel or approaches being parallel to a length direction of a forearm Ar and the clamping part 20 of the surgical instrument 100 is disposed on an axis Oa extending along the length direction of the forearm Ar or approaches the axis Oa. Thereby, swinging of the clamping part 20 is prevented or reduced even when the wrist of the hand holding the surgical instrument 100 rotates.

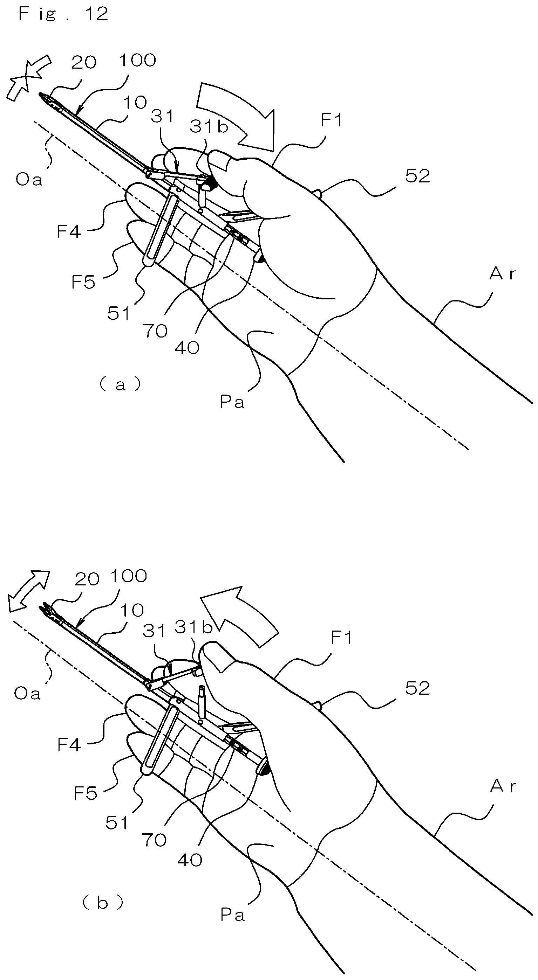

FIG. 12(a) is a schematic view showing an operation of closing the clamping part of the surgical instrument of the present embodiment. FIG. 12(b) is a schematic view showing an operation of opening the clamping part of the surgical instrument of the present embodiment. As shown in FIG. 12(a) and FIG. 12(b), the surgical instrument 100 arranged as described above is held with the contact part 40 being contacted with the palm Pa of the hand, the fingers F4 and F5 (the ring finger and the middle finger) being latched on the one portion 51a of the first support part 51, the other portion 51b of the first support part 51 being sandwiched by the finger F2 (the index finger) and the finger F3 (the middle finger), and the second support part 52 being disposed between a finger F1 (the thumb) and the finger F2. When, in this state, the finger F1 is flexed and straightened and the operating part 31 is reciprocated between the tip end side and the proximal end side of the length direction of the surgical instrument 100, the clamping part 20 opens and closes.

With the surgical instrument 100 in the present embodiment, the clamping part 20, the main body 10, the coupling part 70, and the contact part 40 are coupled successively in one direction and extend in the length direction, the first support part 51 is connected and fixed to the intermediate portion of the main body 10 and extends in the direction intersecting the length direction of the surgical instrument 100, and the surgical instrument 100 is of the substantially cross shape. For this reason, the length direction of the surgical instrument 100 is made parallel or approaches being parallel to the length direction of the forearm Ar and the clamping part 20 is disposed on the axis Oa extending along the length direction of the forearm Ar or approaches the axis Oa when the surgical instrument 100 is held with the contact part 40 being contacted with the palm of the hand and the fingers F1 to F4 being latched on the first support part 51. Consequently, swinging of the clamping part 20 can be prevented or reduced even when the wrist of the hand holding the surgical instrument 100 rotates around the axis Oa (see FIG. 11(b)), and the surgical instrument 100 can be operated easily while viewing an image of an endoscope in comparison to a surgical instrument of a pliers shape or a scissors shape.

In this embodiment, a reciprocation direction of the operating part 31 is the same as the length direction of the surgical instrument 100 since the operating part 31 of the opening/closing mechanism 30 is coupled to the main body and undergoes the reciprocation, which is the pivoting movement around the axis orthogonal to the length direction of the main body 10, between the tip end side and the proximal end side of the length direction of the main body 10. Therefore, swinging of the clamping part 20 due to the reciprocation of the operating part 31 can be suppressed sufficiently by the hand holding the surgical instrument 100 in comparison to a case where the reciprocation direction of the operating part 31 differs from the length direction of the surgical instrument 100.

In the present embodiment, the opening/closing mechanism 30 is provided with the lock mechanism 60 and this lock mechanism 60 is enabled to retain the clamping part 20 in the closed state and to release the retention. Accordingly, the clamping part 20 can be retained in the closed state and the clamping part 20 can be retained in the state of clamping an object such as a needle, etc., without continuing to apply a force to the operating part 31 and operability of the surgical instrument 100 can be improved.

In this embodiment, the lock mechanism 60 has the locking member 61, the opening 62, the narrow part 63, the locking member 61 has the hook Hk at the tip end part and is connected and fixed to the main body 10, the opening 62 and the narrow part 63 are provided in the arm part 31a of the operating part 31, the opening 62 penetrates through the arm part 31a, the narrow part 63 is an elastically deformable small-diameter portion, when the operating part 31 is pivoted to the proximal end side, the tip end part of the locking member 61 is inserted into the opening 62 such that the hook Hk projects to the opposite side (upper surface side) of the operating part 31 with respect to the main body 10 and in this state, when the finger rest part 31b of the operating part 31 is pressed in the width direction, the opening 62 is moved slightly in the width direction by the elastic deformation of the narrow part 63. As a consequence, the hook Hk can be hooked on the edge part of the opening 62 and the hooking of the hook Hk can be released, and locking of the operating part 31 and release of locking of the operating part 31 can be performed easily.

The surgical instrument of the present embodiment is not restricted to just the illustrated example described above and obviously, various modifications can be applied within a scope not deviating from the gist of the present invention. For example, whereas the support parts 50 of the present embodiment are two parts of the first support part 51 and the second support part 52, the support members 50 can be one part of the first support part 51 or can be three or more parts.

Whereas with the present embodiment, the surgical instrument 100 is arranged to be capable of being adjusted to extend and contract in the length direction by successively coupling the coupling part 70 and the contact part 40 to the proximal end part 10b of the main body 10 by screw connection or directly coupling the contact part 40 to the proximal end part 10b of the main body 10, the coupling method is not restricted to screw connection and can be a fitting type joint, etc., or the main body 10 and the contact part 40 can be coupled such as to be capable of extension and contraction by a telescopic method, a slide mechanism, a mechanism such as a turnbuckle, etc., without using the coupling part 70.

Whereas with the present embodiment, the main body 10, the coupling part 70, and the contact part 40 are arranged as separate members, main body 10, the coupling part 70, and the contact part 40 can be formed integrally. The main body 10 and the contact part 40 can be formed integrally when the coupling part 70 is not used. For this reason, it is preventable that the coupling part 70 and/or the contact part 40 detach from the main body 10 and drop into the body of a patient during surgery.

Whereas with the present embodiment, the main body 10 and the support parts 50 (the first support part 51 and the second support part 52) are arranged as separate members, the main body 10 and the support parts 50 can be formed integrally. Accordingly, it is preventable that the support parts 50 detach from the main body 10 and drop into the body of a patient during surgery.

Whereas with the present embodiment, the main body 10 and the locking member 61 of the lock mechanism 60 are arranged as separate members, the main body 10 and the locking member 61 can be formed integrally. For this reason, it is preventable that the locking member 61 detaches from the main body 10 and drops into the body of a patient during surgery.

### Reference Signs List

100 ... surgical instrument, 10 ... main body, 10a ... tip end part, 10b ... proximal end part, 10p, 10q ... slot, 20 ... clamping part, 21 ... pivoting piece, 30 ... opening/closing mechanism, 31 ... operating part, 31a ... arm part, 31b ... finger rest part, 32 ... shaft, 33 ... joint, 40 ... contact part, 40a ... axial part, 40b ... hemispherical part, 50 ... support parts, 51 ... first support part, 51a ... one portion, 51b ... other portion, 52 ... second support part, 60 ... lock mechanism, 61 ... locking member, 62 ... opening, 63 ... narrow part, 70 ... coupling part, Ar ... forearm, F1, F2, F3, F4, F5 ... finger, Hk ... hook, Oa ... axis, P1, P2, P3, P4, P5 ... pin, Pa ... palm of the hand, R1, R2 ... recess part, Sp ... notch, Up, Dw, Tp, Pr, Fr, Bk ... arrow.

## Claims

1. A surgical instrument for holding objects comprising:
a main body extending in one direction,
a clamping part which is placed at a tip end of the main body and which can grasp the objects,
a contact part placed at a proximal end of the main body,
an opening/closing mechanism opening and closing the clamping part, and
support parts extending in one direction, wherein
the main body and each of the support parts are connected and fixed to intersect in a cross shape.

2. The surgical instrument according to claim 1, wherein
the opening/closing mechanism includes an operating part, and
the operating part is connected to the main body, and rotates around an axis perpendicular to a length direction of the main body, and reciprocates between a tip end side and a proximal end side of the length direction of the main body.

3. The surgical instrument according to claim 2, wherein
the clamping part opens when the operating part of the opening/closing mechanism rotates to the tip end side of the main body, and
the clamping part closes when the operating part of the opening/closing mechanism rotates to the proximal end side of the main body.

4. The surgical instrument according to claim 3, wherein
the opening/closing mechanism further includes a lock mechanism, and
the lock mechanism retains the clamping part in a closed state and releases the retention.

5. The surgical instrument according to claim 4, wherein
the lock mechanism retains the operating part of the opening/closing mechanism in a rotated position toward the proximal end side of the main body and releases the retention.

6. The surgical instrument according to one of claims 1-5, wherein
the support parts make of two parts which are a first support part and a second support part, and
the first support part and the second support part are placed at an interval in the length direction of the main body.

7. The surgical instrument according to one of claims 1-5, further comprising:
a coupling part which is arranged such that the coupling part and the contact part can be connected successively to the proximal end of the main body, and such that the contact part can be connected directly to the proximal end of the main body without using the coupling part.

8. The surgical instrument according to one of claims 1-5, wherein
the main body and the contact part are formed integrally.
